# EUROPEAN PATENT APPLICATION

(11) **EP 3 351 629 A1**
(43) Date of publication of application: **25.07.2018**
(21) Application number: 16846543.3
(22) Date of filing: 14.09.2016
(51) Int. Cl.: C12N 15/09, A01K 67/027, C07K 14/47, C07K 14/575, C07K 16/26, C12N 1/15, C12N 1/19, C12N 1/21, C12N 5/10, C12Q 1/02, C12Q 1/68, C12P 21/08

(54) **METHOD FOR IDENTIFYING BIOACTIVE PROTEIN, AND BIOACTIVE PROTEIN OBTAINED BY SAID METHOD**

(30) Priority: 14.09.2015 JP 2015180831
(71) Applicant: Tokyo Institute of Technology, Tokyo 152-8550 (JP); LifeIS INC., Yokohama-shi, Kanagawa 241-0824 (JP)
(72) Inventor: AIZAWA Yasunori, Tokyo 152-8550 (JP); KITANO Shohei, Tokyo 152-8550 (JP); KIDA Yuichiro, Yokohama-shi Kanagawa 241-0824 (JP)
(74) Representative: Gille Hrabal
(86) International application number: PCT/JP2016/077198
(87) International publication number: WO 2017/047672

(57) **Abstract**

An object of the present invention is to provide a method for identifying a physiologically active protein translated from an ORF other than mORF present on mRNA.

The object can be solved by a method for identifying a physiologically active protein, wherein an ORF which encodes a protein having a physiological activity, other than a main open reading frame (ORF) is identified in eukaryotic mRNA, comprising the steps of: (1) introducing an expression vector incorporating a candidate ORF to cells, and culturing the introduced cells, (2) detecting a protein bound to a candidate protein by immunoprecipitating the candidate protein translated from the candidate ORF, from the cultured cells, (3) determining a candidate protein in which another protein bound to the candidate protein is detected as a physiologically active protein.

## Description

### TECHNICAL FIELD

The present invention relates to a method for identifying a physiologically active protein, and a physiologically active protein obtained by the method. According to the present invention, it is possible to identify physiologically active proteins other than the main protein encoded by messenger RNA.

### BACKGROUND ART

Currently, it is believed that messenger RNA (hereinafter referred to as mRNA) transcribed from a eukaryotic protein gene has only one open reading frame (hereinafter referred to as ORF) encoding a functional protein, that is, mRNA is monocistronic. This is one of the major principles of current genetics, as described in comparison with the polycistronic bacterial gene having an operon structure.

However, in the nucleotide sequence of mRNA of a eukaryotic organism, a plurality of ORF regions having a start codon and a stop codon in the same frame exist on one mRNA. In many mRNAs, one ORF among the plural ORFs is significantly longer than the other ORF. Generally, it is believed that the longest ORF encodes a physiologically active protein having a physiological activity and a cell function.

Non-patent literatures 1 and 2 disclose that an ORF (upstream ORF: hereinafter referred to as uORF) having a start codon on an upstream of a main ORF (hereinafter sometimes referred to as mORF) is involved in translation control of mORF. These translated proteins have the function of translational control of mORF, but they do not themselves have physiological activities. That is to say, no protein having physiological activity translated from ORF other than mORF was found from animals.

### CITATION LIST

### PATENT LITERATURE

[NON-PATENT LITERATURE1] Cell, (USA),1986, vol.45, p.201-207
[NON-PATENT LITERATURE2] Proceedings of the National Academy of Sciences of the United States of America, (USA), 2004, vol.101, p.11269-11274
[NON-PATENT LITERATURE3] Nature cell biology, (England), 2007, vol.9, p.660-665
[NON-PATENT LITERATURE4] SCIENCE, (USA), 2013, vol.339, p.1328-1331
[NON-PATENT LITERATURE5] Journal of Cell Biology, (USA), 2012, vol.196, p.801-810

### SUMMARY OF INVENTION

### TECHNICAL PROBLEM

Thus, an object of the present invention is to provide a method for identifying a physiologically active protein translated from an ORF other than mORF present on mRNA and a physiologically active protein obtained by the identification method.

### SOLUTION TO PROBLEM

In order to identify physiologically active proteins translated from ORFs other than mORF present on mRNA, the present inventors incorporated an ORF other than mORF into a vector and tried to express the ORF by introducing the vector into the cells as shown in the examples. However, among the 72 kinds of vectors used, bands of the only 6 kinds of vectors were detected by western blotting. Most proteins were not expressed, although a vector having a strong promoter is used and overexpressed in cells. It was surprising. No physiologically active proteins translated from ORFs other than mORF have been found from animals. The reason for this is considered to be that there is essentially no physiologically active protein translated from ORF other than mORF. According to the present invention, however, it was also found that physiologically active proteins which are translated from ORFs other than mORF also exist in animals. Further, it is difficult to detect proteins translated from ORFs other than mORF. Thus, it is believed that this is one reason why physiologically active proteins translated from ORFs other than mORF were not found.

The present inventors have conducted intensive studies into a method for identifying a physiologically active protein translated from ORFs other than mORF present in mRNA. As a result, the present inventors surprisingly found that physiologically active proteins can be identified by introducing an expression vector incorporating a candidate ORF into cells and detecting a binding of the expressed protein to other proteins in the cells. Further, mice wherein the obtained physiologically active protein is knocked out, were prepared, and behaviors such as anxiety behavior and child abandonment were observed. That is to say, it was confirmed that the obtained physiologically active protein has an influence on the mental activity on the basis of childcare behavior.

The present invention is based on the above findings.

Namely, the present invention relates to:
[1] a method for identifying a physiologically active protein, wherein an ORF which encodes a protein having a physiological activity, other than a main open reading frame (ORF) is identified in eukaryotic mRNA, comprising the steps of: (1) introducing an expression vector incorporating a candidate ORF to cells, and culturing the introduced cells, wherein a start codon of the candidate ORF is AUG or non-AUG in which any one base of AUG is different from AUG, (2) detecting a protein bound to a candidate protein translated from the candidate ORF, from the cultured cells, (3) determining a candidate protein in which another protein bound to the candidate protein is detected as a physiologically active protein,
[2] the method for identifying a physiologically active protein of the item [1], wherein a proteasome inhibitor or a lysosome inhibitor is added to a cell culture medium in the cell culture step (1),
[3] the method for identifying a physiologically active protein of the item [1] or [2], further comprising a step of:
   detecting an expression of the candidate protein translated from a candidate ORF in advance and selecting a candidate ORF having a large amount of expression, before the binding protein detection step (2),
[4] the method for identifying a physiologically active protein of any one of the items [1] to [3], wherein the candidate ORF satisfies one or more conditions selected from the group consisting of: (a) the start codon of the candidate ORF is present on the 5 'UTR side from the start codon of the main ORF, (b) a protein encoded by a candidate ORF is 10 amino acids or more, (c) a candidate ORF is an ORF other than the main ORF in mRNA of one eukaryotic organism, and an amino acid sequence translated from the candidate ORF has 50% or more identity to an amino acid sequence translated from an ORF other than the main ORF in mRNA of one or more other eukaryotic organisms, (d) the start codon of the candidate ORF is AUG; and (e) peptides expressed in a cell of the eukaryotic organism are analyzed by mass analysis, and an ORF of an amino acid sequence obtained by using the molecular weight of the obtained peptide is a matched one retrieved from a database of the candidate ORFs,
[5] the method for identifying a physiologically active protein of any one of the items [1] to [4], wherein a method for detecting a binding protein in the binding protein detection step (2) is selected from the group consisting of an immunoprecipitation method, yeast two-hybrid method, a protein array method, a label method using peroxidase, and a BioID method,
[6] the method for identifying a physiologically active protein of any one of the items [1] to [5], wherein the eukaryotic organism is a mammal,
[7] (1) a protein consisting of an amino acid sequence of SEQ ID NO:1, (2) a protein comprising an amino acid sequence of SEQ ID NO:1, and exhibiting a function of suppressing abnormality of mental activity on the basis of childcare behavior, (3) a protein consisting of an amino acid sequence in which one or plural amino acids are deleted, substituted, inserted and/or added at one or plural positions in the amino acid sequence of SEQ ID NO:1, and exhibiting a function of suppressing abnormality of mental activity on the basis of childcare behavior, or (4) a protein comprising an amino acid sequence in which one or plural amino acids are deleted, substituted, inserted and/or added at one or plural positions in the amino acid sequence of SEQ ID NO:1, and exhibiting a function of suppressing an abnormality of mental activity on the basis of childcare behavior,
[8] (1) a protein consisting of an amino acid sequence of SEQ ID NO:10 or 11, (2) a protein comprising an amino acid sequence of SEQ ID NO: 10 or 11, and having a binding ability with the peroxiredoxin 1, (3) a protein consisting of an amino acid sequence in which one or plural amino acids are deleted, substituted, inserted and/or added at one or plural positions in the amino acid sequence of SEQ ID NO:10 or 11, and having a binding ability with the peroxiredoxin 1, or (4) a protein comprising an amino acid sequence in which one or plural amino acids are deleted, substituted, inserted and/or added at one or plural positions in the amino acid sequence of SEQ ID NO:10 or 11, and having a binding ability with the peroxiredoxin 1,
[9] (1) a protein consisting of an amino acid sequence of SEQ ID NO:14 or 15, (2) a protein comprising an amino acid sequence of SEQ ID NO: 14 or 15, and having a binding ability with the Q Subcomponent Binding Protein, (3) a protein consisting of an amino acid sequence in which one or plural amino acids are deleted, substituted, inserted and/or added at one or plural positions in the amino acid sequence of SEQ ID NO: 14 or 15, and having a binding ability with the Q Subcomponent Binding Protein, or (4) a protein comprising an amino acid sequence in which one or plural amino acids are deleted, substituted, inserted and/or added at one or plural positions in the amino acid sequence of SEQ ID NO: 14 or 15, and having a binding ability with the Q Subcomponent Binding Protein,
[10] a polynucleotide encoding the protein of any one of the items [7] to [9],
[11] an expression vector comprising the polynucleotide of the item [10],
[12] a transformant comprising the polynucleotide of the item 10],
[13] an antibody or a fragment thereof, which binds to the protein of any one of the items claims [7] to [9],
[14] a knockout non-human animal or cell, wherein an expression of a gene encoding the protein of the item [7] is partially or completely suppressed, and
[15] a method for screening a compound that suppresses abnormality of mental activity on the basis of childcare behavior, characterized by administering a candidate compound to the knockout non-human animal or cell of the item claim [14].

Kondoh et al. reported that peptides (pri) having physiological activity of very short 11 or 32 amino acids are expressed in flies (Non-Patent literature 3). However, peptides of 11 amino acids and 32 amino acids are peptides having the same function. Therefore, this report does not indicate that one polynucleotide has a protein translated from a main open reading frame (ORF) and an ORF encoding a peptide having a different physiological activity.

### ADVANTAGEOUS EFFECTS OF INVENTION

According to the method for identifying physiologically active proteins of the present invention, it is possible to identify physiologically active proteins encoded by ORFs other than mORF of mRNAs that have not been considered to have physiological activity in the past. For example, the physiologically active protein obtained by the identification method of the present invention is thought to be a protein related to behavior and can be used for the development of medicine for mental diseases. Further, a knockout mouse of the physiologically active protein can be used as a model mouse for mental diseases. Furthermore, a compound for preventing or treating a mental disorder on the basis of childcare behavior can be screened by administering a candidate drug to the knockout mouse.

### BRIEF DESCRIPTION OF THE DRAWINGS

FIG. 1 is a diagram showing ORFs other than mORF included in an mRNA.
FIG. 2 is a view showing a scheme for identifying an ORF encoding a physiologically active protein from the candidate ORFs in Example 1.
FIG. 3 is a schematic view of an N-terminal FLAG-tagged uORF expression vector and a C-terminal FLAG-tagged uORF expression vector (A), and a photograph showing the expression (Western blotting) of these vectors in HEK 293 T cells (B). Expression in HEK 293 T cells shows only 26 ORFs.

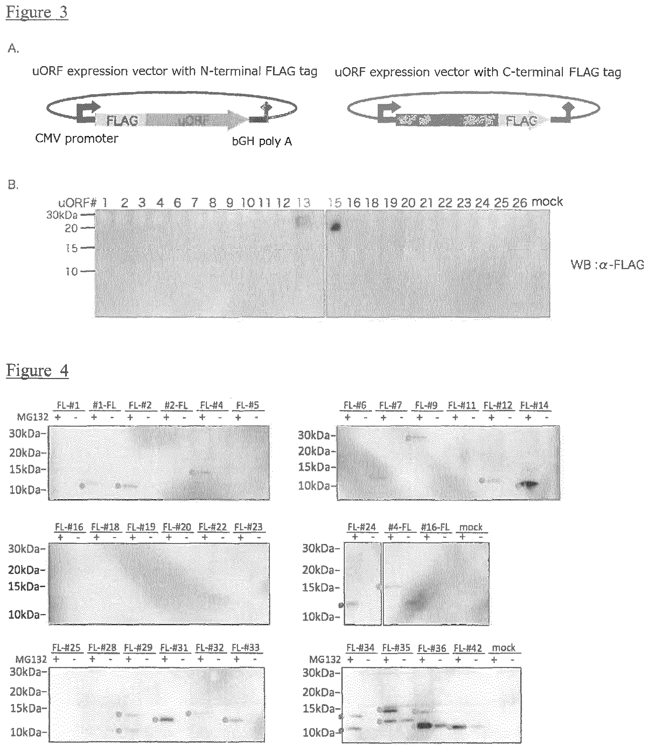

FIG. 3 is a schematic view of a N-terminal FLAG-tagged uORF expression vector and C-terminal FLAG-tagged uORF expression vector (A), and a photograph showing the expression (Western blotting) of these vectors in HEK 293 T cells (B). Expression in HEK 293 T cells shows only 26 ORFs.
FIG. 4 is a photograph showing that the expression of LAG tagged uORF is observed by western blotting, after adding a proteasome inhibitor (MG132) to the cell culture medium

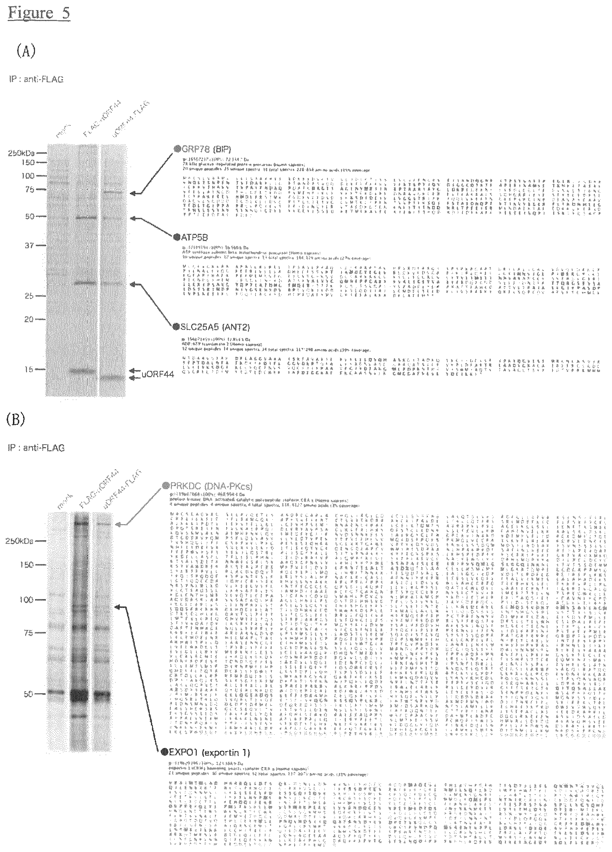

FIG. 5 is photographs showing that intracellular proteins binding to uORF44p are detected by immunoprecipitation of HEK293T cells transfected with the vector containing uORF44 with an anti-FLAG antibody and western blotting, and a view showing sequences of the binding proteins.

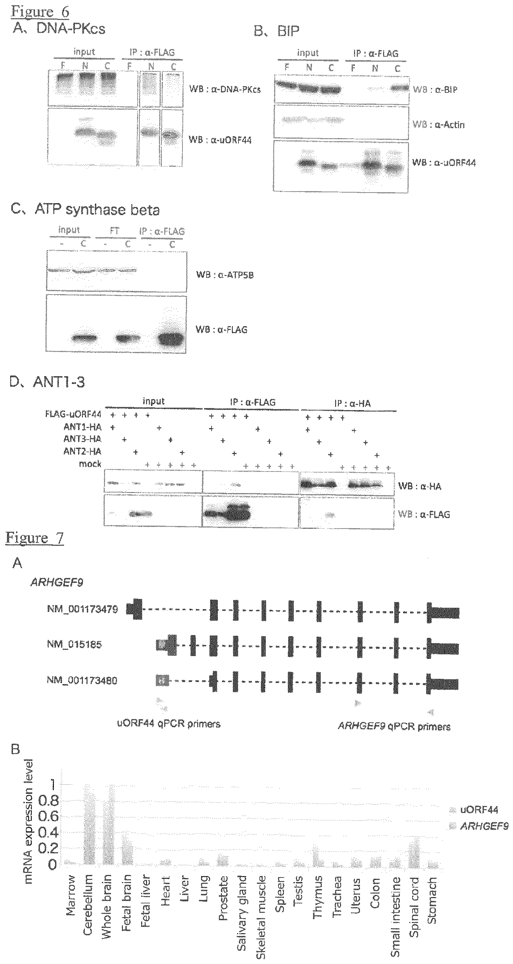

FIG. 6 is photographs of western blotting showing that immunoprecipitation is performed with antibodies against candidate proteins binding to uORF44p and the binding between uORF44p and each protein is observed.
FIG. 7 is a graph showing that expressions of three mRNA variants of ARHGEF9 containing uORF44 are detected by qPCR for 20 human organ cDNAs.

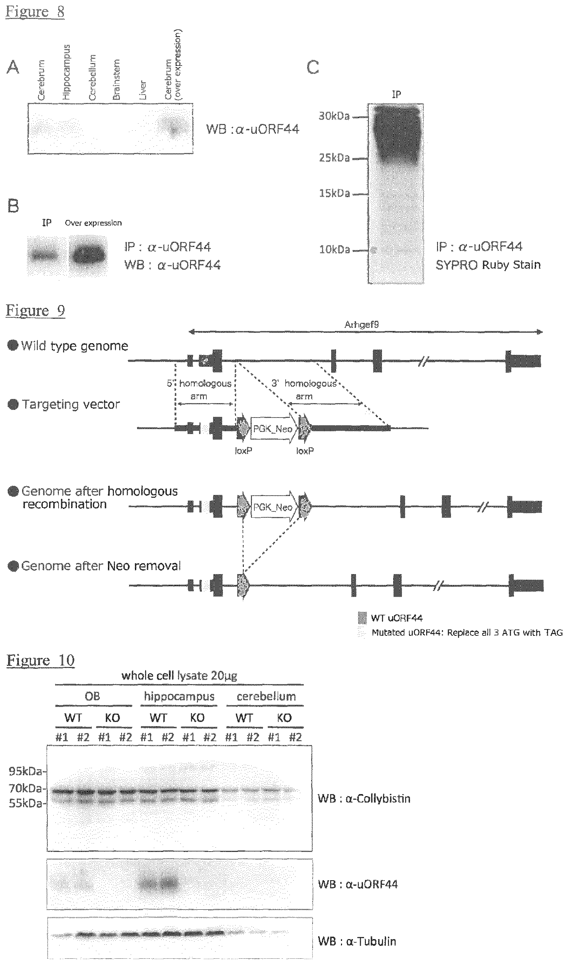

FIG. 8 is photographs showing that expressions of uORF44p in cerebrum, cerebellum, hippocampus, brainstem, and liver are detected by western blotting.
FIG. 9 is a schematic view showing a targeting vector for preparing a knockout mouse.
FIG. 10 is photographs showing that translations of uORF44 and Arhgef9 in a uORF44 knockout mouse are detected by western blotting.

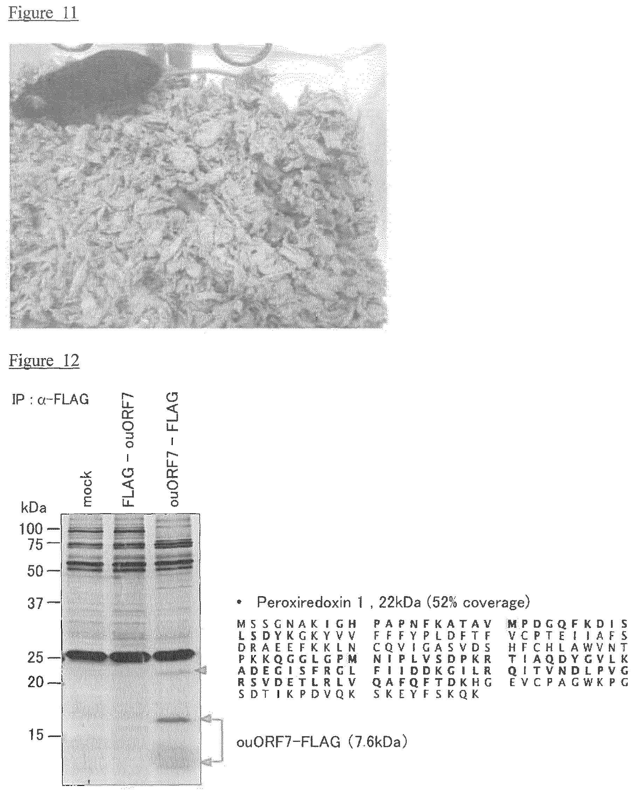

FIG. 11 is a photograph showing that the uORF44 knockout mouse does not show a maternal behavior (retrieving and crouching over).
FIG. 12 is a photograph showing that cells into which a vector containing ouORF7 has been introduced are immunoprecipitated with an anti-FLAG antibody and an intracellular protein binding to ouORF7p is detected by western blotting, and a view showing a sequence of the binding protein..

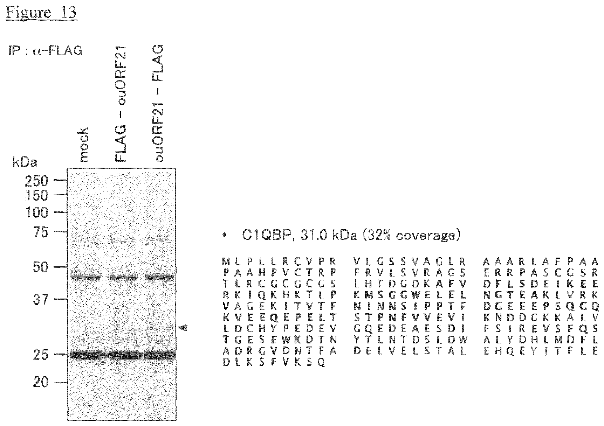

FIG. 13 is a photograph showing that cells into which a vector containing ouORF21p has been introduced are immunoprecipitated with an anti-FLAG antibody and an intracellular protein binding to ouORF21p is detected by western blotting, and a view showing a sequence of the binding protein.

### DESCRIPTION OF EMBODIMENTS

### [1] Method for identifying physiologically active protein

### [1] Method for identifying physiologically active protein

The method for identifying a physiologically active protein of the present invention is characterized in that an ORF which encodes a protein having a physiological activity, other than a main open reading frame (ORF) is identified in eukaryotic mRNA, and comprises the steps of: (1) introducing an expression vector incorporating a candidate ORF to cells, and culturing the introduced cells, wherein a start codon of the candidate ORF is AUG or non-AUG in which any one base of AUG is different from AUG, (2) detecting a protein bound to a candidate protein translated from the candidate ORF, from the cultured cells, (3) determining a candidate protein in which another protein bound to the candidate protein is detected as a physiologically active protein.

### <<Open reading frame encoding physiologically active protein>>

The physiologically active protein identified by the method for identifying a physiologically active protein of the present invention is not a protein encoded by a main ORF (hereinafter referred to as mORF) present in eukaryotic mRNA, but a protein encoded by an ORF other than mORF. That is, target ORFs identified by the identification method of the present invention means an ORF other than mORF.

Eukaryotic mRNA is thought to be a monocistronic mRNA with one mORF encoding a physiologically active protein, as compared to a bacterial gene having polycistronic mRNA containing two or more ORFs. The eukaryotic mORF is often the longest ORF in its mRNA (sometimes referred to as the longest ORF). However, the target ORF in the present invention is often relatively short, and may be referred to as the "ORF other than the longest ORF."

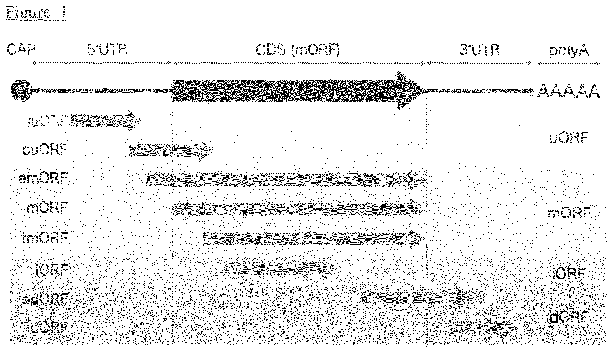

FIG. 1 shows target ORFs classified according to the positional relationship with mORF. In the present specification, an ORF having an open reading frame different from that of mORF and having a start codon upstream of the start codon of mORF is referred to as upstream URF (hereinafter referred to as uORF). Further, an ORF having an open reading frame different from mORF and having a start codon downstream of the start codon and a stop codon downstream of the stop codon of mORF is referred to as downstream URF (hereinafter referred to as dORF).

The target ORF of the present invention can be classified into the following 7 ORFs with respect to mORF encoding the protein shown by the thick arrow in FIG. 1:
(1) an uORF having a start codon and a stop codon present in the 5 'UTR and not overlapping with mORF (isolated uORF: hereinafter referred to as iuORF),
(2) an uORF having a start codon in the 5 'UTR and a stop codon in mORF, and overlapping with mORF (overlapping uORF: hereinafter referred to as ouORF),
(3) an ORF having a start codon in 5 'UTR, and having the same reading frame as mORF, and extending mORF towards the 5' end side (extended mORF: hereinafter referred to as emORF),
(4) an ORF having a start codon within mORF, and having the same reading frame as mORF, and shortening the 5 'end side of mORF (truncated mORF: hereinafter referred to as tmORF),
(5) an ORF having a start codon and a stop codon present in the mORF (included ORF: hereinafter referred to as iORF),
(6) a dORF having a start codon within mORF and a stop codon in 3'UTR, and overlapping with mORF (overlapping dORF: hereinafter referred to as odORF), and
(7) a dORF having a start codon and a stop codon present in the 3'UTR (isolated dORF: hereinafter referred to as idORF).

In the identification method of the present invention, it is identified that the protein encoded by the target ORF has a physiological activity. The wording "having physiological activity" as used herein is not limited, as long as it is involved in a physiological function in vivo. For example, a translational regulation of mORF may be regarded as physiological activity like the proteins described in non-patent literatures 1 and 2. However, it is preferable that the protein itself is involved in a physiological function in vivo.

### (Candidate ORF)

The candidate ORF identified by the identification method of the present invention is an ORF other than mORF present in the mRNA. The minimum size of the candidate ORF is not limited, but may be 6 base lengths or more (start codon + stop codon). In the case where a protein has 5 amino acids or more, it may be 18 bases or more. In the case where a protein has 10 amino acids or more, it may be 33 bases or more. In the case where a protein has 30 amino acids or more, it may be 93 bases or more. Further, ORFs starting from different start codons, but ending with the same stop codon are preferably considered as different candidate ORFs.

It is possible to prepare a database of candidate ORFs and to preliminarily select candidate ORFs by using the database of candidate ORFs. The database of candidate ORFs can be created by using an mRNA database. The database of mRNA to be used is not particularly limited, but it can be obtained from, for example, a UCSC Genome Browser. The mORF is specified from the sequence of the obtained mRNA and the above-mentioned iuORF, ouORF, emORF, tmORF, iORF, odORF, and idORF can be extracted, and then a database can be created.

Further, the start codon of each ORF may be only AUG (ATG), but it is possible to use a non-AUG (ATG) start codon. The non-AUG start codon is a sequence in which one base of AUG is replaced with another base. Specifically, there may be mentioned UUG (TTG), GUG (GTG), CUG (CTG), AAG, AGG, ACG, AUA (ATA), AUU (ATT), and AUC (ATC). That is to say, an ORF starting from one or more start codons selected from the group consisting of AUG, UUG, GUG, CUG, AAG, AGG, ACG, AUA, AUU, and AUC can be the candidate ORF.

### (Eukaryote)

A eukaryote (eukaryotic organism) having the candidate ORF of the present invention is not particularly limited, but includes an animal, a plant, a fungus, or a protist. A eukaryote is characterized by having a cell nucleus in the cell.

As the animal, there may be mentioned vertebrate animals and invertebrate animals. As the vertebrate animal, for example, there may be mentioned mammals, birds, reptiles, amphibians, teleost fish, cartilage fish, myxiniformes, lampreys, and the like, and mammals are preferable. Mammals include monotremata, marsupialia, edentata, dermoptera, chiroptera, carnivora, insectivora, proboscidea, perissodactyla, artiodactyla, tubulidentata, squamata, sirenia, subordo cetacea, primates, rodentia, and lagomorpha. More specifically, there may be mentioned human, bovine, ovine, goat, swine, camel, deer, horse, rabbit, dog, cat, ferret, buffalo, donkey, ferocus, reindeer, mouse, rat, hamster, squirrel, and monkey. As the invertebrate animal, for example, there may be mentioned crustacean, diplopoda, pauropoda, chilopoda, symphyla, insecta, and the like.

In the identification method of the present invention, ORFs other than mORF present in these eukaryotic mRNAs can be used as candidate ORFs.

### <<Cell culture step (1)>>

The cell culture step (1) is a step in which an expression vector incorporating a candidate ORF is introduced into a cell and the introduced cell is cultured.

### (Expression vector)

The expression vector used in the identification method of the present invention is not limited as long as it has a construction capable of expressing the incorporated gene in the cell. That is, there may be mentioned a vector obtained by inserting a candidate ORF into a known expression vector appropriately selected according to the cell to be used. The expression vector to be used can be constructed on the basis of an autonomously replicating vector (such as a plasmid), which exists as an extrachromosomal entity and whose replication is not dependent on chromosomal replication.

In the identification method of the present invention, the vector is preferably a vector capable of tagging a protein translated from a candidate ORF. Many candidate ORFs are not confirmed to be expressed in cells, and thus antibodies to most of these candidate ORFs have not been obtained. In the identification method of the present invention, it is necessary to confirm by western blotting that the candidate ORF is expressed in the cell into which the vector is introduced. Further, it is necessary to confirm by immunoprecipitation that the protein translated from the candidate ORF binds to other proteins in the cell. In that case, western blotting or immunoprecipitation can be performed by using antibodies against the tag without using antibodies to the candidate ORF.

A length of the tag is not particularly limited as long as the method for identifying a physiologically active protein of the present invention may be carried out, and, for example, a tag having a length of 500 amino acids or less can be used. However, the length of the tag is preferably 2 to 50 amino acids, more preferably 3 to 30 amino acids, further preferably 4 to 20 amino acids, and most preferably 5 to 10 amino acids. Tags with long amino acid lengths can be used, but in the case of long tags, tags may sometimes bind to proteins in cells and cause nonspecific reactions. Further, the long tags may strongly express most candidate proteins, and thus it is difficult to compare the intracellular expression of the candidate proteins themselves. Therefore, the length of the tag is preferably relatively short.

As the tag, a tag used in this field can be used. For example, a FLAG tag, HA tag, HIS tag, myc tag, TAP tag, or the like can be used.

In order to express the candidate ORF by introducing it into the cell, the expression vector preferably contains, in addition to the sequence of the candidate ORF, a DNA sequence that regulates its expression and a genetic marker for selecting a cell and the like. DNA sequences regulating expression comprises promoters, terminators, DNA sequences encoding signal peptides, and the like. The genetic marker may be appropriately selected according to the method of selection of the transformant. For example, a gene encoding drug resistance or a gene encoding auxotrophy can be used.

### (Cell)

Cells into which the expression vector is introduced are not particularly limited, but eukaryotic cells are preferably used. In particular, in order to identify normal physiological activities, cells derived from the same species as the species of the candidate ORF are preferable. For example, when a candidate ORF derived from a human is to be expressed, cells derived from a human are preferably used. For example, however, it is possible to identify physiologically active proteins by introducing candidate ORFs derived from humans into cells derived from mice.

Examples of the cells include HEK 293 T cells, HepG 2 cells, or A 549 cells. Further, ES cells, iPS cells, or differentiating cells thereof can be used.

Furthermore, when the obtained physiologically active protein is related to various diseases, cells to be screened for drugs can be appropriately selected depending on the disease.

### (Proteasome inhibitor and lysosome inhibitor)

A proteasome inhibitor can be added to the cell culture medium in the cell culture step (1). It can be suppressed that the protein translated from the ORF is degraded by the proteasome by adding the proteasome inhibitor. Many candidate ORFs are relatively short proteins of 100 amino acids or less, and thus the candidate ORFs are thought to be difficult to form a stable structure. Therefore, some candidate ORFs are degraded by proteasome. In the case of such a candidate ORF, it is possible to stably express the translated candidate protein from the ORF by adding a proteasome inhibitor.

The proteasome inhibitor is not particularly limited but, for example, MG 132 can be mentioned.

Furthermore, in the cell culture step (1), a lysosome inhibitor can be added to the cell culture medium. It can be suppressed that the protein translated from the ORF is degraded by the lysosome by adding the proteasome inhibitor. As the lysosome inhibitor, chloroquine can be mentioned.

### <<Protein detection step(2)>>

In the protein detection step (2) of the present invention, a protein that binds to the candidate protein translated from the candidate ORF is detected from cultured cells.

A method for detecting intracellular proteins binding to the candidate protein is not particularly limited, as long as the binding of two proteins can be detected by methods which are normally used in the technical field. As a specific method, there may be mentioned an immunoprecipitation method, yeast two-hybrid method, a protein array method, a label method using peroxidase (such as, an APEX method using ascorbic acid peroxidase), or a BioID method.

### (Immunoprecipitation)

Immunoprecipitation can be carried out by methods known in this field. The antibodies used can be obtained by immunizing rabbits and the like with proteins (polypeptides) translated from the candidate ORFs. However, it is not efficient to prepare each antibody for each of the many candidate proteins. Therefore, it is preferable to use a vector capable of tagging the candidate protein as an expression vector, and to immunoprecipitate with an antibody against the tag. As the tag antibody, there may be mentioned an anti-FLAG tag antibody, an anti-HA tag antibody, an anti-HIS tag antibody, an anti-myc tag antibody, or an anti-TAP tag antibody.

A protein encoded by a candidate ORF capable of binding intracellular proteins can be detected by analyzing the precipitate obtained by immunoprecipitation by western blotting.

### (Yeast two-hybrid method)

The yeast two-hybrid method can be carried out by a method known in this field. Specifically, the candidate protein is fused to the DNA binding domain (DBD) of GAL4 and the intracellular protein is fused to the activator domain of GAL, and whereby it is possible to detect a protein encoded by a candidate ORF capable of binding intracellular proteins. E. coli can be used instead of yeast. Further, LexA, or the Ras signaling pathway can be used instead of GAL4.

### (Protein array method)

The protein array method can be carried out by a method known in this field. Specifically, a protein encoded by a candidate ORF capable of binding intracellular proteins can be detected by immobilizing intracellular proteins on protein arrays and bringing candidate proteins into contact therewith. Conversely, by immobilizing the candidate protein on the protein array and bringing the intracellular protein into contact therewith, it is possible to detect the protein encoded by the candidate ORF capable of binding the intracellular protein.

### (Label method using peroxidase)

An APEX method using ascorbic acid peroxidase can be mentioned as an example of a labeling method using peroxidase. The APEX method uses ascorbic acid peroxidase as the tag, as reported in Non Patent Literature 4. Specifically, when ascorbic acid peroxidase or an improved peroxidase thereof is expressed intracellularly in a form fused with a protein of interest, other proteins existing in the vicinity thereof can be labeled with biotin or the like. In this method, therefore, by specifically recovering and analyzing only the labeled protein after cell lysis, other proteins with which the protein of interest interacts within the cell, can be identified.

### (BioID method)

As reported in Non Patent Literature 5, BirA is used in the BioID method. Specifically, a candidate protein and BirA are expressed as a fusion protein and brought into contact with a binding protein in a cell, whereby it is possible to detect a protein encoded by a candidate ORF capable of binding intracellular proteins.

### <<Determination step (3)>>

In the determination step (3), a candidate protein in which another protein bound to the candidate protein is detected, is determined as a physiologically active protein.

Proteins with physiological activity often exhibit physiological activity by interacting with other molecules (proteins) in vivo. In other words, candidate proteins that bind to other molecules (proteins) in vivo are thought to have physiological activity. Therefore, in the protein detection step (2), a candidate protein confirmed to bind to other proteins in the cell can be determined as a physiologically active protein.

### <<Step for detecting expression of candidate protein>>

The identification method of the present invention may include a step for preliminarily detecting the expression of the candidate protein that is translated from the candidate ORF before the binding protein detection step (2) (hereinafter referred to as a preliminary detection step). Candidate proteins with stable expression can be selected by including the preliminary detection step, and the candidate proteins to be immunoprecipitated in the protein detection step (2) can be selected. The expression level of the candidate protein is not particularly limited as long as it can be detected. For example, it is not limited so long as it can be detected by a western blotting method, but is preferably 1ag/g or more, more preferably 10ag/g or more. More particularly, in the western blotting method, an expression level capable of applying 10 pg or more of protein to the gel as one band is preferable.

In the preliminary detection step, the proteasome inhibitor may be added to the cell culture medium. It is possible to stabilize the expression of candidate proteins that may be degraded by the proteasome by adding the proteasome inhibitor.

The method for detecting the expression of the candidate protein includes but is not limited to, for example, a method using an antibody or mass spectrometry. As the method using an antibody, a western blotting method, a dot blotting method or the like can be used.

### <<Selection of candidate ORFs>>

In the identification method of the present invention, the candidate ORFs can be preliminarily selected. The candidate ORF satisfying one or more conditions selected from the group consisting of:
(a) the start codon of the candidate ORF is present on the 5 'UTR side from the start codon of the main ORF,
(b) a protein encoded by a candidate ORF is 10 amino acids or more,
(c) a candidate ORF is an ORF other than the main ORF in mRNA of one eukaryotic organism, and an amino acid sequence translated from the candidate ORF has 50% or more identity to an amino acid sequence translated from an ORF other than the main ORF in mRNA of one or more other eukaryotic organisms,
(d) the start codon of the candidate ORF is AUG; and
(e) peptides expressed in a cell of the eukaryotic organism are analyzed by mass analysis, and an ORF of an amino acid sequence obtained by using the molecular weight of the obtained peptide is a matched one retrieved from a database of the candidate ORFs; may be selected. One of these conditions (a) to (e) may be applied, or two or more of them may be applied in combination. Further, in the respective conditions (a) to (e), it is also possible to select various criteria.

### (a) Position of candidate ORF

Regarding positions of the candidate ORFs, for example, a candidate ORF in which the start codon thereof exists on the 5 'UTR side from the start codon of the main ORF, may be selected. As described above, candidate ORFs other than mORF can be classified into seven types, i.e. iuORF, ouORF, emORF, tmORF, iORF, odORF, and idORF. The present inventor has found that a selection pressure is applied to uORF such as iuORF and ouORF rather than dORF such as odORF and idORF. That is to say, it is also considered that the protein translated from uORF is more likely to have physiological activity than dORF. Thus, the possibility of identifying physiologically active proteins may be increased, for example, by selecting iuORF and/or ouORF from candidate ORFs classified into the seven types.

However, it is also possible to identify proteins with different physiological activities by selecting either emORF, tmORF, iORF, odORF, or idORF. Therefore, one or more ORFs selected from the group consisting of iuORF, ouORF, emORF, tmORF, iORF, odORF, and idORF can be selected.

### (b) Length of candidate ORF

Regarding the length of the candidate ORF, for example, ORFs with a length of protein encoded by a candidate ORF of 10 amino acids or more may be selected. The minimum size of the candidate ORF used in the present invention may be, for example, 6 bases in length (initiation codon + stop codon) or more. Therefore, the length of the candidate ORF is not limited, but preferably 10 or more amino acids, more preferably 20 or more amino acids, more preferably 30 or more amino acids, more preferably 40 or more amino acids. However, short proteins may exhibit special physiological activities, and thus, proteins of 10 to 20 amino acids may be selected, proteins of 10 to 30 amino acids may be selected, proteins of 10 to 40 amino acids may be selected, proteins of 30 to 40 amino acids can be selected.

### (c) Identity of candidate ORF

Regarding the identity of the candidate ORF, an ORF in which the candidate ORF is an ORF other than the main ORF in mRNA of one eukaryotic organism, and the amino acid sequence translated from the candidate ORF has 50% or more identity to an amino acid sequence translated from an ORF other than the main ORF in mRNA of one or more other eukaryotic organisms, may be selected. The candidate ORF used in the present invention is a eukaryotic ORF. When genetic sequences or amino acid sequences of two animal species are compared, a protein having physiological activity is likely to be generally conserved among several organism species. Therefore, by selecting ORFs conserved in two or more species, the possibility of identifying biologically active proteins increases. For example, the identity is preferably 50% or more, more preferably 60% or more, further preferably 70% or more, further preferably 80% or more, most preferably 90% or more. The species to be compared is not particularly limited, and any eukaryotic organism can be selected. Further, the number of species to be compared is not limited.

As a method of determining identity, software (pairwise alignment tool) usually used in this field can be used. The identity can be calculated using, for example, Needle (EMBL-EBI).

### (d) Start codon of candidate ORF

Regarding the start codon of the candidate ORF, an ORF having the start codon of AUG (ATG) can be selected. As mentioned above, an AUG (ATG) or a non-AUG (ATG) start codon can be used as the start codon of the candidate ORF. Generally, AUG is frequently used as a start codon, but a non-AUG start codon may be used in some cases. Therefore, it is preferable that the start codon of the candidate ORF is AUG (ATG), but it is also possible to select one or more ORFs having the start codon selected from the group consisting of AUG, UUG, GUG, CUG, AAG, AGG, ACG, AUA, AUU, and AUC. It is possible to identify novel physiologically active proteins by using the non-AUG (ATG) start codon

### (e) Detection of intracellular candidate ORF by mass spectrometry

In the identification method of the present invention, a candidate ORF can be selected by searching the amino acid sequence of the peptide detected in the cell by mass spectrometry and the amino acid sequence of the candidate ORF. The amino acids constituting the peptide can be identified by mass spectrometry. Further, candidate ORFs containing the detected peptides can be identified by comparing the composition of the amino acids constituting the peptide with the amino acid sequence in the database of candidate ORFs.

It is possible to select a candidate ORF with high probability of being expressed in the cells by using the mass spectrometric detection. It is also possible to select a candidate protein with low conservation in two or more eukaryotes.

Cells used for mass spectrometry are not particularly limited as long as they are eukaryotic cells. Cells of tissues or organs can be used instead of cultured cells. Depending on the type of cell used, it is considered that the detected peptides are different, and thus various candidate proteins can be selected.

### [2] Physiologically active protein

### (2-1) Mental activity-related protein

An anxiety controlling protein of the present invention is (1) a protein consisting of an amino acid sequence of SEQ ID NO:1, (2) a protein comprising an amino acid sequence of SEQ ID NO:1, and exhibiting a function of suppressing abnormality of mental activity on the basis of childcare behavior, (3) a protein consisting of an amino acid sequence in which one or plural amino acids are deleted, substituted, inserted and/or added at one or plural positions in the amino acid sequence of SEQ ID NO:1, and exhibiting a function of suppressing abnormality of mental activity on the basis of childcare behavior (hereinafter sometimes referred to as a "functionally equivalent variation"), or (4) a protein comprising an amino acid sequence in which one or plural amino acids are deleted, substituted, inserted and/or added at one or plural positions in the amino acid sequence of SEQ ID NO:1, and exhibiting a function of suppressing abnormality of mental activity on the basis of childcare behavior. The protein (4) of the present invention contains the functionally equivalent variation (protein (3)).

The amino acid sequence of human and mouse mental activity-related protein is the same sequence shown in SEQ ID NO: 1. The human nucleic acid sequence is shown in SEQ ID NO: 2 and the mouse nucleic acid sequence is shown in SEQ ID NO: 3.

In humans, the mental activity-related protein of the invention is a protein in which an ORF is present in the 5 'UTR of mORF of ARHGEF 9. An mRNA encoding the mental activity-related protein is broadly expressed in the nervous system tissues such as the cerebellum, the whole brain, and the spinal cord. In addition, the mental activity-related protein is expressed in a mouse's cerebrum and hippocampus.

On the other hand, the mORF of ARHGEF9 is expressed in many neural tissues other than the cerebrum and hippocampus, and the distribution of the expression of uORF44 protein and ARHGEF9 protein does not seem to be perfectly matched.

The "functionally equivalent variation" of the present invention is not limited, as long as it is a protein consisting of an amino acid sequence in which 1 to 10, preferably 1 to 7, more preferably 1 to 5 (such as 1 to several) amino acids are deleted, substituted, inserted, and/or added at one or plural positions in the amino acid sequence of SEQ ID NO:1, and exhibiting a function of suppressing abnormality of childcare behavior. Further, its origins are not limited to humans.

The functionally equivalent variation of the present invention includes not only a human mutation of the protein consisting of the amino acid sequence of SEQ ID NO: 1, but also functionally equivalent variations derived from organisms other than humans (e.g., human, mouse, rat, hamster, or dog). Further, it includes a protein which is prepared by using a polynucleotide artificially modified by genetic engineering based on polynucleotides encoding those natural polypeptides (i.e., mutants derived from humans or functionally equivalent variations derived from organisms other than humans), or based on a polynucleotide encoding the protein consisting of the amino acid sequence of SEQ ID NO:1, or the like.

The wording "abnormality of mental activity on the basis of childcare behavior" in this specification includes, for example, "abnormality of childcare behavior" or "child abandonment." In the present specification, therefore, a presentation of the "function of suppressing abnormality of mental activity on the basis of child care behavior" can be confirmed by observing that mice expressing the mental activity-related protein of the present invention exhibit normal child-rearing behavior. More specifically, when a mouse expressing a functionally equivalent variation or the like into which a mutation is introduced does not show the abnormality of childcare behavior, like a mouse expressing a wild type anxiety controlling protein, it can be judged to exhibit the "function of suppressing abnormality of mental activity on the basis of child care behavior."

Further, the protein of the present invention can also be prepared by genetic engineering. Specifically, appropriate primers or probes are designed on the basis of information on the base sequence of the polynucleotide encoding the protein consisting of the amino acid sequence of SEQ ID NO:1. Then, a polymerase chain reaction (PCR) method or a hybridization method is carried out using the primers or probes, and a sample (such as total RNA or mRNA fraction, cDNA library, or phage library) derived from an organism of interest (for example, a mammal (such as bovine, human, mouse, rat, hamster, or dog)), and whereby a polynucleotide encoding the protein is obtained. The desired protein can be obtained by confirming that the protein encoded by the polynucleotide exhibits the "function of suppressing abnormality of childcare behavior."

The "functionally equivalent variation" of the present invention includes a protein prepared by using a polynucleotide artificially modified by genetic engineering based on information on the base sequence of a polynucleotide encoding the protein consisting of the amino acid sequence of SEQ ID NO: 1. Specifically, a polynucleotide is obtained by a conventional method, such as site-specific mutagenesis, and then the polynucleotide is expressed using an appropriate expression system. Then, the desired protein can be obtained by confirming that the expressed protein exhibits, for example, "a function of suppressing abnormality of mental activity on the basis of child care behavior."

In addition, the protein of the present invention can also be prepared by chemical synthesis methods. The chemical synthesis of peptides includes a liquid phase method and a solid phase method, and the solid phase method is preferable. As the solid phase method, there may be mentioned an Fmoc solid phase synthesis method, a Boc solid phase synthesis method. The synthesized peptides can be purified by a known method such as high performance liquid chromatography (HPLC).

### (2-2) Peroxiredoxin binding protein

A peroxiredoxin binding protein of the present invention is (1) a protein consisting of an amino acid sequence of SEQ ID NO:10 or 11, (2) a protein comprising an amino acid sequence of SEQ ID NO: 10 or 11, and having a binding ability with the peroxiredoxin 1, (3) a protein consisting of an amino acid sequence in which one or plural amino acids are deleted, substituted, inserted and/or added at one or plural positions in the amino acid sequence of SEQ ID NO:10 or 11, and having a binding ability with the peroxiredoxin 1, or (4) a protein comprising an amino acid sequence in which one or plural amino acids are deleted, substituted, inserted and/or added at one or plural positions in the amino acid sequence of SEQ ID NO:10 or 11, and having a binding ability with the peroxiredoxin 1.

The amino acid sequences of the human and mouse peroxiredoxin binding proteins are the same sequences shown in SEQ ID NOs: 10 and 11. Further, the human nucleic acid sequence thereof is shown in SEQ ID NO: 12, and the mouse nucleic acid sequence thereof is shown in SEQ ID NO: 13. The mouse peroxiredoxin binding protein also has the binding ability with the peroxiredoxin 1.

The peroxiredoxin binding protein of the present invention is a protein in which an ORF is present in the 5 'UTR of mORF of RELL 2 (RELT-like 2) in humans. Peroxiredoxin (Prdx) is an enzyme that reduces reactive oxygen species such as hydrogen peroxide and alkyl hydroperoxide, and may respond to heat shock and oxidative stress.

The " functionally equivalent variation" of the present invention is not limited, as long as it is a protein consisting of an amino acid sequence in which 1 to 10, preferably 1 to 7, more preferably 1 to 5 (such as 1 to several) amino acids are deleted, substituted, inserted, and/or added at one or plural positions in the amino acid sequence of SEQ ID NO:10 or 11, and having the binding ability with the peroxiredoxin. Further, its origins are not limited to humans.

The "functionally equivalent variation" of the present invention is not limited, as long as it is a protein consisting of an amino acid sequence in which 1 to 10, preferably 1 to 7, more preferably 1 to 5 (such as 1 to several) amino acids are deleted, substituted, inserted, and/or added at one or plural positions in the amino acid sequence of SEQ ID NO:10 or 11, and having the binding ability with the peroxiredoxin. Furhther, its origins are not limited to humans.

The functionally equivalent variation of the present invention includes not only a human mutant of the protein consisting of the amino acid sequence of SEQ ID NO: 10 or 11, but also functionally equivalent variations derived from organisms other than humans (e.g., human, mouse, rat, hamster, or dog). Further, it includes a protein which is prepared by using a polynucleotide artificially modified by genetic engineering based on polynucleotides encoding those natural polypeptides (i.e., mutant derived from humans or functionally equivalent variations derived from organisms other than humans), or based on a polynucleotide encoding the protein consisting of the amino acid sequence of SEQ ID NO:10 or 11, or the like.

The functionally equivalent variation of the present invention includes not only human mutant of the protein consisting of the amino acid sequence of SEQ ID NO: 10 or 11, but also variations functionally equivalent derived from organismes other than human (eg, human, mouse, rat, hamster, or dog). Further, it includes a protein which is prepraed by using a polynucleotide artificially modified by genetic engineering based on polynucleotides encoding those natural polypeptides (i.e., mutants derived from humans or variations functionally equivalent derived from organisms other than humans), or based on a polynucleotide encoding the protein consisting of the amino acid sequence of SEQ ID NO:10 or 11, or the like.

Further, the protein of the present invention can also be prepared by genetic engineering. Specifically, appropriate primers or probes are designed on the basis of information on the base sequence of the polynucleotide encoding the protein consisting of the amino acid sequence of SEQ ID NO:10 or 11. Then, a polymerase chain reaction (PCR) method or a hybridization method is carried out using the primers or probes, and a sample (such as total RNA or mRNA fraction, cDNA library, or phage library) derived from an organism of interest (for example, a mammal (such as bovine, human, mouse, rat, hamster, or dog)), and whereby a polynucleotide encoding the protein is obtained. The desired protein can be obtained by confirming that the protein encoded by the polynucleotide exhibits the binding ability with the peroxiredoxin.

The " functionally equivalent variation" of the present invention includes a protein prepared by using a polynucleotide artificially modified by genetic engineering based on information on the base sequence of a polynucleotide encoding the protein consisting of the amino acid sequence of SEQ ID NO: 10 or 11. Specifically, a polynucleotide is obtained by a conventional method, such as site-specific mutagenesis, and then the polynucleotide is expressed using an appropriate expression system. Then, the desired protein can be obtained by confirming that the expressed protein exhibits, for example, the binding ability with the peroxiredoxin.

In addition, the protein of the present invention can also be prepared by chemical synthesis methods. The chemical synthesis of peptides includes a liquid phase method and a solid phase method, and the solid phase method is preferable. As the solid phase method, there may be mentioned an Fmoc solid phase synthesis method, a Boc solid phase synthesis method. The synthesized peptides can be purified by a known method such as high performance liquid chromatography (HPLC).

### (2-3) C1QBP binding protein

A Q Subcomponent Binding Protein (hereinafter, sometimes referred to as a C1QBP) binding protein of the present invention is (1) a protein consisting of an amino acid sequence of SEQ ID NO:14 or 15, (2) a protein comprising an amino acid sequence of SEQ ID NO: 14 or 15, and having a binding ability with the Q Subcomponent Binding Protein, (3) a protein consisting of an amino acid sequence in which one or plural amino acids are deleted, substituted, inserted and/or added at one or plural positions in the amino acid sequence of SEQ ID NO: 14 or 15, and having a binding ability with the Complement Component 1 and/or the Q Subcomponent Binding Protein, or (4) a protein comprising an amino acid sequence in which one or plural amino acids are deleted, substituted, inserted and/or added at one or plural positions in the amino acid sequence of SEQ ID NO: 14 or 15, and having a binding ability with the Complement Component 1 and/or the Q Subcomponent Binding Protein.

The amino acid sequences of the human and mouse C1QBP binding protein are the same sequences shown in SEQ ID NOs: 14 and 15. Further, the human nucleic acid sequence thereof is shown in SEQ ID NO: 16, and the mouse nucleic acid sequence thereof is shown in SEQ ID NO: 17. The mouse C1QBP binding protein also has the binding ability with the C1QBP.

The C1QBP binding protein of the present invention is a protein in which an ORF is present in the 5 'UTR of mORF of FBXL 5 (F-box and leucine - rich repeat protein 5) in the human. The C1QBP is localized in mitochondria, and suppresses cell death caused by oxidative stress.

. The "functionally equivalent variation" of the present invention is not limited, as long as it is a protein consisting of an amino acid sequence in which 1 to 10, preferably 1 to 7, more preferably 1 to 5 (such as 1 to several) amino acids are deleted, substituted, inserted, and/or added at one or plural positions in the amino acid sequence of SEQ ID NO:14 or 15, and having the binding ability with the C1QBP. Further, its origins are not limited to humans.

The functionally equivalent variation of the present invention includes not only a human mutation of the protein consisting of the amino acid sequence of SEQ ID NO: 14 or 15, but also functionally equivalent variations derived from organisms other than human (e.g., human, mouse, rat, hamster, or dog). Further, it includes a protein which is prepared by using a polynucleotide artificially modified by genetic engineering based on polynucleotides encoding those natural polypeptides (i.e., mutants derived from humans or functionally equivalent variations derived from organisms other than humans), or based on a polynucleotide encoding the protein consisting of the amino acid sequence of SEQ ID NO:14 or 15, or the like.

Further, the protein of the present invention can also be prepared by genetic engineering. Specifically, appropriate primers or probes are designed on the basis of information on the base sequence of the polynucleotide encoding the protein consisting of the amino acid sequence of SEQ ID NO:14 or 15. Then, a polymerase chain reaction (PCR) method or a hybridization method is carried out using the primers or probes, and a sample (such as total RNA or mRNA fraction, cDNA library, or phage library) derived from an organism of interest (for example, a mammal (such as bovine, human, mouse, rat, hamster, or dog)), and whereby a polynucleotide encoding the protein is obtained. The desired protein can be obtained by confirming that the protein encoded by the polynucleotide exhibits the binding ability with the C1QBP.

The " functionally equivalent variation" of the present invention includes a protein prepared by using a polynucleotide artificially modified by genetic engineering based on information on the base sequence of a polynucleotide encoding the protein consisting of the amino acid sequence of SEQ ID NO: 14 or 15. Specifically, a polynucleotide is obtained by a conventional method, such as site-specific mutagenesis, and then the polynucleotide is expressed using an appropriate expression system. Then, the desired protein can be obtained by confirming that the expressed protein exhibits, for example, the binding ability with the C1QBP.

In addition, the protein of the present invention can also be prepared by chemical synthesis methods. The chemical synthesis of peptides includes a liquid phase method and a solid phase method, and the solid phase method is preferable. As the solid phase method, there may be mentioned an Fmoc solid phase synthesis method, a Boc solid phase synthesis method. The synthesized peptides can be purified by a known method such as high performance liquid chromatography (HPLC).

### [3] Polynucleotide

The polynucleotide of the present invention is not particularly limited, as long as it encodes the protein of the present invention. For example, there may be mentioned a polynucleotide consisting of the base sequence of SEQ ID NO: 1, or a polynucleotide comprising the base sequence of SEQ ID NO: 1. The term "polynucleotide" in the present specification includes DNA and RNA.

The method for preparing the polynucleotide of the present invention is not particularly limited, but for example, includes (1) a method using PCR, (2) a conventional genetic engineering method (that is, a method of selecting a transformant containing a cDNA of interest from a transformant transformed with a cDNA library), or (3) a chemical synthesis method, or the like. Each preparing method will be sequentially explained below.

### [4] Expression vector and transformant of the present invention

The Expression vector of the present invention contains the polynucleotide of the present invention. That is, the vector of the present invention is not particularly limited as long as it includes the polynucleotide according to the present invention. For example, there may be mentioned a vector obtained by inserting the polynucleotide of the present invention into a known expression vector appropriately selected according to the host cell to be used. The expression vector of the present invention can be constructed on the basis of a self-replicating vector (such as a plasmid), which exists as an extrachromosomal element and can replicate independently of the replication of chromosomes. Alternatively, the expression vector of the present invention may be a vector which is integrated into the genome of the host microorganism and replicated together with chromosomes, when the host is transformed with the vector. The construction of the vector of the present invention can be carried out by ordinary procedures or methods commonly used in genetic engineering.

To express a protein having a desired activity by transforming a host microorganism with the expression vector of the present invention, it is preferable that the expression vector contains, for example, a polynucleotide capable of controlling the expression, or a genetic marker to select transformants, in addition to the polynucleotide of the present invention. As the polynucleotide capable of controlling the expression, for example, a promoter, a terminator, or a polynucleotide encoding a signal peptide for secretion, may be used in the present invention. The promoter which can be used in the present invention is not particularly limited, so long as it shows a transcriptional activity in a host microorganism. The promoter can be obtained as a polynucleotide which controls the expression of a gene encoding a protein the same as or different from that derived from the host microorganism. The signal peptide is not particularly limited, so long as it contributes to the protein secretion in a host microorganism. The signal peptide can be obtained as a polynucleotide derived from a gene encoding a protein same as or different from that derived from the host microorganism. The genetic marker can be appropriately selected in accordance with the method for selecting a transformant. As the genetic marker, for example, a drug resistance gene or a gene complementing an auxotrophic mutation can be used in the present invention.

According to the present invention, a cell transformed with the expression vector is provided. A host-vector system which can be used in the present invention is not particularly limited. For example, a system utilizing E. coli, Actinomycetes, yeasts, filamentous fungi, or cells of eukaryote, or a system for the expression of a fusion protein using such a microorganism, can be used.

Transformation of cells with the expression vector can be carried out in accordance with an ordinary method.

In the present invention, the transformant of the present invention is cultured, and the resulting transformant or culture is used to obtain the protein of the present invention. According to another embodiment of the present invention, the process for producing the novel protein of the present invention can be provided. Cultivation of the transformant (including culturing conditions) can be carried out in a fashion substantially similar to that of the original cells used to prepare the transformant. As the method for recovering the protein of interest after the cultivation of the transformant, commonly used procedures can be used.

In the eukaryotic host cells, for example, cells of vertebrates, insects, and yeast are included. As the vertebral cell, there may be mentioned, for example, a COS cell as a simian cell, a Chinese hamster ovary cell (CHO), a human embryonic kidney-derived HEK293 cell, or a 293T cell derived from a human.

As an expression vector for a vertebral cell, a vector containing a promoter positioned upstream of the polynucleotide to be expressed, an RNA splicing site, a polyadenylation site, a transcription termination sequence, and the like may be generally used. The vector may further contain a replication origin, if necessary. As the expression vector, there may be mentioned, for example, pSV2dhfr containing an SV40 early promoter, pEF-BOS containing a human elongation factor promoter, or pCEP4 containing a cytomegalovirus promoter (Invitrogen).

The expression vector may be incorporated into cells by, for example, a DEAE-dextran method, a calcium phosphate-DNA co-precipitation method, a method using a commercially available transfection reagent, or an electroporation method.

The transformant of the present invention may be cultured in accordance with the conventional method, and the protein of the present invention is produced on the cell surface. As a medium to be used in the culturing, a medium commonly used in a desired host cell may be appropriately selected. In the case of the 293-EBNA cell, a medium such as a Dulbecco's modified Eagle's minimum essential medium (DMEM) with a serum component such as fetal bovine serum (FBS) and G418 may be used.

The protein produced within the cell by culturing the transformants may be separated and purified therefrom by various known separation techniques making use of the physical properties, chemical properties and the like of the protein. More particularly, the protein of the present invention may be purified by treating a cell extract containing the protein of the present invention with a commonly used treatment, for example, a treatment with a protein precipitant, ultrafiltration, various liquid chromatography techniques such as molecular sieve chromatography (gel filtration), adsorption chromatography, ion exchange chromatography, affinity chromatography, or high performance liquid chromatography (HPLC), or dialysis, or a combination thereof.

### [5] Antibody

An antibody, such as a polyclonal antibody or a monoclonal antibody, which reacts with the protein of the present invention, may be obtained by directly administering the protein of the present invention or a fragment thereof to various animals. Alternatively, it may be obtained by a DNA vaccine method (Raz, E. et al., Proc. Natl. Acad. Sci. USA, 91, 9519-9523, 1994; or Donnelly, J. J. et al., J. Infect. Dis., 173, 314-320, 1996), using a plasmid into which a polynucleotide encoding the protein of the present invention is inserted.

The polyclonal antibody may be produced from a serum or eggs of an animal such as a rabbit, a rat, a goat, or a chicken, in which the animal is immunized and sensitized by the protein of the present invention or a fragment thereof emulsified in an appropriate adjuvant (for example, Freund's complete adjuvant) by intraperitoneal, subcutaneous, or intravenous administration. The polyclonal antibody may be separated and purified from the resulting serum or eggs in accordance with conventional methods for protein isolation and purification. Examples of the separation and purification methods include, for example, centrifugal separation, dialysis, salting-out with ammonium sulfate, or a chromatographic technique using DEAE-cellulose, hydroxyapatite, protein A agarose, and the like.

The monoclonal antibody may be easily produced by those skilled in the art, according to, for example, a cell fusion method of Kohler and Milstein (Kohler, G. and Milstein, C., Nature, 256, 495-497, 1975).

A mouse is immunized intraperitoneally, subcutaneously, or intravenously several times at an interval of a few weeks by a repeated inoculation of emulsions in which the protein of the present invention or a fragment thereof is emulsified into a suitable adjuvant such as Freund's complete adjuvant. Spleen cells are removed after the final immunization, and then fused with myeloma cells to prepare hybridomas.

As a myeloma cell for obtaining a hybridoma, a myeloma cell having a marker such as a deficiency in hypoxanthine-guanine phosphoribosyltransferase or thymidine kinase (for example, mouse myeloma cell line P3X63Ag8.U1) may be used. As a fusing agent, polyethylene glycol may be used. As a medium for preparation of hybridomas, for example, a commonly used medium such as an Eagle's minimum essential medium, a Dulbecco's modified minimum essential medium, or an RPMI-1640 medium may be used by adding properly 10 to 30% of a fetal bovine serum. The fused strains may be selected by a HAT selection method. A culture supernatant of the hybridomas is screened by a well-known method such as an ELISA method or an immunohistological method, to select hybridoma clones secreting the antibody of interest. The monoclonality of the selected hybridoma is guaranteed by repeating subcloning by a limiting dilution method. Antibodies in an amount which may be purified are produced by culturing the resulting hybridomas in a medium for 2 to 4 days, or in the peritoneal cavity of a pristane-pretreated BALB/c strain mouse for 10 to 20 days.

The resulting monoclonal antibodies in the culture supernatant or the ascites may be separated and purified by conventional polypeptide isolation and purification methods. Examples of the separation and purification methods include, for example, centrifugal separation, dialysis, salting-out with ammonium sulfate, or chromatographic technique using DEAE-cellulose, hydroxyapatite, protein A agarose, and the like.

Further, the monoclonal antibodies or the antibody fragments containing a part thereof may be produced by inserting the whole or a part of a gene encoding the monoclonal antibody into an expression vector and introducing the resulting expression vector into appropriate host cells (such as E. coli, yeast, or animal cells).

Antibody fragments comprising an active part of the antibody such as F(ab')2, Fab, Fab', or Fv may be obtained by a conventional method, for example, by digesting the separated and purified antibodies (including polyclonal antibodies and monoclonal antibodies) with a protease such as pepsin, papain, and the like, and separating and purifying the resulting fragments by standard polypeptide isolation and purification methods.

Further, an antibody which reacts to the protein of the present invention may be obtained in a form of single chain Fv or Fab in accordance with a method of Clackson et al. or a method of Zebedee et al. (Clackson, T. et al., Nature, 352, 624-628, 1991; or Zebedee, S. et al., Proc. Natl. Acad. Sci. USA, 89, 3175-3179, 1992). Furthermore, a humanized antibody may be obtained by immunizing a transgenic mouse in which mouse antibody genes are substituted with human antibody genes (Lonberg, N. et al., Nature, 368, 856-859, 1994).

### [6] Knockout non-human animals and cells of the present invention

The knockout non-human animal of the present invention is not particularly limited as long as the expression of the gene encoding the protein of the present invention is partially or completely suppressed, and it can be prepared by known methods.

For example, in an embryonic stem cell of a target non-human animal such as a cow, a sheep, a goat, a pig, a horse, a mouse, or a chicken, the gene encoding the protein of the present invention on the chromosome is deactivated by a known method of homologous recombination (Nature,326,6110,295(1987);or Cell,51,3,503(1987)), or a mutant clone in which the gene encoding the protein of the present invention on the chromosome is replaced with an arbitrary sequence is prepared (Nature, 350, 6315, 243 (1991)), by using a recombinant vector containing the polynucleotide of the present invention. A chimeric individual composed of an embryonic stem cell clone and a normal cell can be prepared by an injection chimeric method in which the cell is injected into a blastocyst of the fertilized egg of the animal, an aggregation chimera method, or the like, using the mutant clones of embryonic stem cells. Individuals having any mutation in the gene encoding the protein of the present invention present on the chromosome of the cells throughout the body can be obtained by multiplying the chimeric individuals and the normal individuals. Further, by multiplying the individuals, the knockout non-human animal can be obtained as an individual in which the expression of the gene encoding the protein of the present invention is partially or completely suppressed, from homozygotes with mutations in both homologous chromosomes.

Further, a cell of the present invention in which the expression of the gene encoding the protein of the present invention is partially or completely inhibited can be obtained from the knockout non-human animal.

Alternatively, an expression of a cellular endogenous anxiety controlling protein can be suppressed by performing, for example, RNA interference method (for example, Nature, 411, 494-498, 2002) on cells of interest (such as HEK 293 cell derived from human fetal kidney, 293-EBNA cell wherein EBNA-1 gene of Epstein-Barr virus was introduced into the HEK 293 T cell, or 293 T cell derived from human). The resulting cells are also included in the scope of the present invention.

Further, it is also possible to prepare the knockout non-human animal by introducing a mutation into any position of the gene encoding the protein of the present invention on the chromosome. For example, it is also possible to modify the activity of the gene product by introducing a mutation (such as substitution, deletion, and / or insertion of a base(s)) into a translated region of the gene encoding the protein of the present invention on the chromosome. The target gene in the knockout mouse of the present invention is a gene encoded by an ORF other than mORF in mRNA. Therefore, in order to analyze a function of the target gene, it is preferable not to affect the expression of the mORF. Thus, it is preferable to suppress the expression of the target gene by mutating ATG, which is the start codon of the target gene. In the Examples, the expression of the target gene was suppressed by mutating ATG, which is the start codon of uORF 44, to TAG which is a stop codon, and further mutating two ATGs contained in uORF 44 to TAG. However, a method of suppression of gene expression is not limited to the above mutation, but the expression of the target gene can also be suppressed by mutating ATG to other codons.

Further, for example, it is also possible to modify a degree of expression, expression timing, and/or a tissue specificity of expression, etc., by introducing a similar mutation into an expression control region thereof. Furthermore, it is also possible to more actively control the expression timing, an expression site, and/or an expression level etc., by combining with a Cre-loxP system. As such examples, it is known that a target gene was deleted only in that region using a promoter expressed in a specific region of the brain (Cell, 87, 7, 1317 (1996)), or a target gene is organ-specifically deleted at a target time using an adenovirus expressing Cre (Science, 278, 5335(1997)).

Therefore, regarding the gene encoding the protein of the present invention on the chromosome, the expression thereof can be controlled at any time or any tissues as described above, and further, the knockout non-human animal having any insertion, deletion, and/or substitution in the translation region or the expression-regulating region thereof can be prepared. The knockout non-human animal can induce symptoms of various diseases caused by the protein of the present invention at any time, any degree, and/or at any site. In this manner, the knockout non-human animal of the present invention is an extremely useful animal model in the treatment and prevention of various diseases caused by the protein of the present invention.

### [7] Method for screening a compound that suppresses abnormality of mental activity on the basis of childcare behavior

A method for screening a compound that suppresses abnormality of mental activity on the basis of childcare behavior, characterized by administering a candidate compound to the knockout non-human animal or cell according to claim 14.

The method for screening a compound of the present invention is characterized by administering a candidate compound to the knockout non-human animal or cell.

Substances to be tested to which may be applied the screening method of the present invention are not particularly limited, but there may be mentioned, for example, various known compounds (including peptides) registered in chemical files, compounds obtained by combinatorial chemistry techniques (Terrett, N. K. et al., Tetrahedron, 51, 8135-8137, 1995) or normal synthesis techniques, random peptides prepared by employing a phage display method (Felici, F. et al., J. Mol. Biol., 222, 301-310, 1991), siRNA, or antibodies against uORF44 gene or uORF44 protein. In addition, culture supernatants of microorganisms, natural components derived from plants or marine organisms, or animal tissue extracts may be used as the test substances for screening. Further, compounds (including peptides) obtained by chemically or biologically modifying compounds (including peptides) selected by the screening method of the present invention may be used.

In the screening method of the present invention, the test substance can be screened by bringing the test substance into contact with the knockout non-human animal or cell and analyzing a change in the response of the knockout non-human animal or cell.

Furthermore, the present invention may include screened compounds obtained by the screening method and medicines comprising the screened compounds.

### EXAMPLES

The present invention will now be further illustrated by, but is by no means limited to, the following Examples.

### <<Example 1>>

In this example, the uORFs encoding the functional proteins of human mRNA were identified.

### (1) Acquisition of ORF

A FASTA file describing the sequence of 5 'UTR only, the sequence of 5' UTR + mORF and the sequence of 5 'UTR + mORF + 3' UTR in RefSeq Gene of human and mouse was obtained from the UCSC Genome Browser, and the positions of mORFs on mRNA were determined based on this information (human_refseq_5UTR_20131228.fasta, human_refseq_5UTR_CDS_20131228.fasta, human_refseq_5UTR_CDS_3UTR_20131228.fasta). Starting positions of mORFs were determined from the length of 5 'UTR, and end positions of mORFs were determined from the length of 5' UTR + mORF.

Sequence data obtained from the Genome Browser contains duplicated genes and genes without 5 'UTR, and thus the duplicated genes and the genes without 5 'UTR were excluded previously by a program of our own making (oneline_fasta.pl, get_NM.pl, and remove_gene_duplication.pl).

Next, ORFs were extracted by a program of our own making (find_all_ORF.pl). Multiple ORFs having different start codons but identical stop codons were extracted as separate ones.

We obtained the lengths of the ORFs using a program to calculate the length of the ORF (length_orf.pl). The lengths of the ORFs were statistically analyzed by the R language after obtaining the lengths thereof.

### (2) Conversion of uORF to amino acid sequence and acquisition of uORF with 30 amino residues or more

uORFs having the start codon ATG on the 5 'UTR side from the start codon ATG of mORF were extracted from the ORFs other than the obtained human and mouse mORF.

The FASTA files of all human and mouse uORF were changed to amino acid sequences by a translation.pl., and then, only the proteins with a length of 30 amino acid residues or more were obtained by a length.pl.

### (3) Extraction of human uORF and mouse uORF with high identity

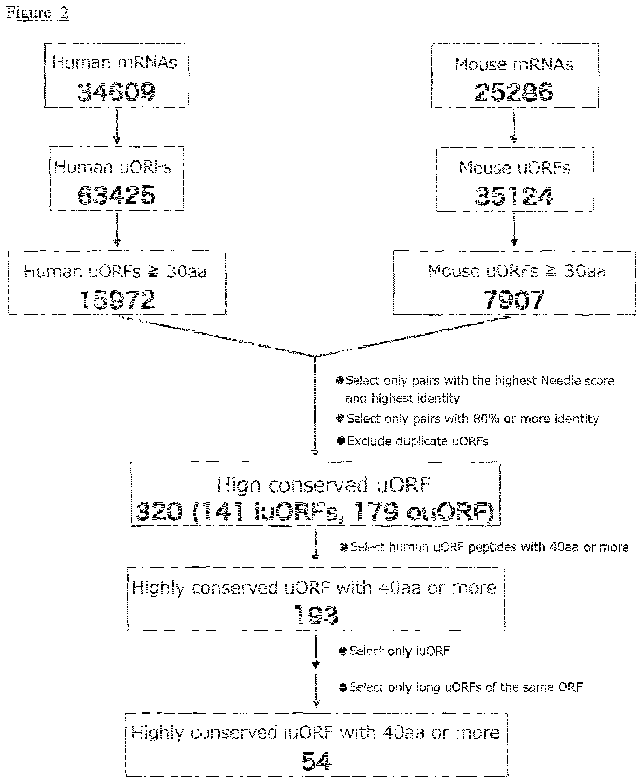

Conservability of human uORF and mouse uORF was calculated using a pairwise alignment tool (Needle). A pairwise alignment of one human uORF protein and all mouse uORF proteins with Needle was obtained and a mouse uORF protein having the highest score and the highest identity was obtained. Then, homolog pairs were prepared by a program (called needle 30. PI) for performing the above calculation for all human uORFs. Since uORFs with the same sequence are included therein, one duplicate sequence was removed by remove_dup_seq.pl, and finally uORF pairs having 40 amino acid residues or more and an identity of 80% or more were extracted by Microsoft Excel. In addition, ouORFs overlapping with mORFs were excluded therefrom. FIG. 2 shows a scheme for the identification of candidate ORFs encoding functional proteins.

As a result of the above identification, 54 highly conserved human uORFs could be extracted from 63,425 uORFs.

### (4) Construction of expression plasmid

FLAG tagged expression plasmids were constructed in order to immunoprecipitate the resulting 54 ORF-encoded proteins. 108 plasmids' construction was attempted for expressing the uORF protein fused with the FLAG tag at the N-terminus or C-terminus of 54 uORF (FIG. 3A).

PcDNA3_FLAG-insert, pcDNA3_insert-FLAG, pcDNA3_HA-insert, and pcDNA3_insert-HA were used for preparation of the FLAG tagged expression plasmid and the HA tagged expression plasmid. The ORF sequence was inserted into the vector by PCR (Takara Prime Star GXL polymerase), restriction enzyme treatment (using any two of NEB, NheI-HF, KpnI-HF, AgeI-HF, BamHI-HF, EcoRI-HF, XbaI), and ligation (Wako 2×ligation mix).

72 kinds of expression plasmids can be constructed.

### (5) Western blotting

The resulting 72 expression plasmids were transfected into HEK293T and expressions thereof were examined. HEK 293T was seeded on a culture dish (BD Falcon) coated with poly-L-Lysine (Sigma, QJ-P 4832) on the day before transfection so that confluency at transfection was about 80%. Expression plasmid was mixed with Opt-MEM (Life Technologies) and FuGENE-HD (Promega), and transfected into HEK 239T. In the experiment to confirm the expression, the cells were dissolved in a 1×SDS sample buffer (58.3mM Tris-HCl pH6.8, 1.7%SDS, 5% Glycerol, Complete Protease Inhibitor Cocktail EDTA free (Roche)) after 48 hours of transfection. The proteins were denatured at 95 °C for 5 minutes after sonication.

The proteins were electrophoresed by SDS-PAGE and were transferred to Immobilon P^{sq} (Millipore) or Immobilon P. The membrane was blocked with 5% skim milk (Wako)-PBST (blocking buffer), and reacted with an anti-FLAG antibody (Sigma, F 3165, multiples of 5000) as a primary antibody at room temperature for 1 hour. After completion of the primary antibody reaction, it was washed three times with PBST (0.1% Tween-20) at room temperature for 5 minutes. In the secondary antibody reaction, a secondary antibody (anti mouse IgG antibody (GE, NA 931 V, 5000 fold) was diluted with a blocking buffer and reacted at room temperature for 1 hour. After completion of the secondary antibody reaction, it was washed two times with PBST at room temperature for 5 minutes. Immobilon Western Chemiluminescent HRP Substrate (Millipore) was used as an HRP detection reagent.

As a result, six kinds of uORF proteins can be confirmed as visible bands.

### (6) Suppression of protein by proteasome

It was considered that the uORF protein, in which the expression thereof could not be confirmed, may be degraded by the proteasome. Therefore, it was confirmed whether the expression of the uORF protein was improved using a proteasome inhibitor. 28 plasmids out of 72 plasmids were examined.

The procedure described in paragraph "(5) western blotting" was repeated except that the medium was exchanged into a DMEM containing 10 µM MG 132 (Calbiochem, # 474790) at 24 hours after transfection.

As a result, expressions of 14 FLAG-tagged uORFs among 28 uORFs were confirmed by the western blotting (Figure 4). That is, expressions of half of the 28 uORFs could be confirmed by adding a proteasome inhibitor.

### (7) Immunoprecipitation

Six kinds of uORF stably expressed were immunoprecipitated and it was investigated as to whether they were bound to other proteins in the cell.

The expression vector was transfected into HEK 293 T cells cultured in a 10 cm culture dish using Opt-MEM (Life Technologies) and FuGENE-HD (Promega). After 48 hours of transfection, the cells were washed with PBS and dissolved using 1 mL of lysis buffer (20 m Tris-HCl pH 7.5, 150 mM NaCl, 0.5% NP 40, 1 × Complete EDTA free (Roche) 10 mM Glycerol 2-phosphate, 1 mM NaVO₄) and allowed to stand on ice for 20 minutes. Thereafter, the cells were centrifuged at 4°C, 21,130×g for 30 minutes, and the supernatant was used for immunoprecipitation. 50 µL of anti-DYKDDDDK antibody beads (Wako, 016-22784) or 50 µL of Monoclonal Anti-HA-Agarose (Sigma, A 2095) was used in the immunoprecipitation. The supernatant was mixed with antibody beads lysate, and slowly mixed at 4°C for 1 hour. After completion of the antigen-antibody reaction, it was washed 3 times with 1 mL of a lysis buffer, and then the protein was eluted with 50 µL 1×SDS sample buffer. The eluted sample was subjected to electrophoresis by SDS-PAGE followed by western blotting or SYPRO Ruby staining. The western blotting was performed as described in paragraph "(5) Western blotting." The SYPRO Ruby staining was performed using SYPRO Ruby protein gel stain (Life Technologies, S 12000), in accordance with a product manual.

As a result, it was suggested that a protein (hereinafter referred to as uORF44p) derived from one uORF (hereinafter referred to as uORF44) binds to five kinds of proteins with different molecular weights (FIGS. 5A and 5B).

### (8) Analysis of binding protein

These five binding protein candidates were excised from the SDS-PAGE gel and proteins were identified by mass spectrometry. These proteins are shown in Table 1.

**[Table 1]**

| Protein | Gene | Molecular weight | GI number | Main localization | Main function |
|---|---|---|---|---|---|
| DNK-PKcs | PRKDC | 469kDa | gil119607088 | intranuclear | DNA repair |
| Exportin-1 | EXPO1 | 123kDa | gil119620399 | nuclear membrane | nuclear export |
| BIP | GRP78 | 72kDa | gil16507237 | endoplasmic reticulum | ER stress detection |
| ATP synthase beta | ATP5B | 57kDa | gil32189394 | mitochondrial membrane | ATP synthesis |
| ANT | | 33kDa | gil156071459 | mitochondrial membrane | ATP/ADP transport and apoptosis control |

In order to confirm the results of the immunoprecipitation experiment, western blotting was carried out using each antibody against each binding protein candidate. The procedure described in paragraph "(5) western blotting" was repeated except for using an anti-BIP antibody (Abcam, ab21685, 1000-fold), anti-DNA-PKcs antibody (Abcam, ab1832, 1000-fold), anti-ATPB antibody (Abcam, ab14730, 2000-fold) or anti-HA antibody (Santa Cruz, sc-805, 2000-fold) as a primary antibody. In connection with this, no analysis is done for exportin-1. For ANT, antibodies that can distinguish three kinds of subfamilies (ANT1-3) present in the human ANT gene were not commercially available. Therefore, expression vectors for ANT1-HA, ANT2-HA, and ANT3-HA in which an HA tag was fused to each subtype, were prepared, respectively. Then, after coexpression with the FLAG-tagged uORF44 expression vector, immunoprecipitation with an antibody against one tag was carried out, and western blotting with an antibody against the other tag was carried out.

As shown in Figure 6, it was confirmed that DNA-PKcs and BIP bind to uORF44p. However, ATP synthase beta did not bind uORF44p. Binding of ANT2 and uORF44p was suggested from the results. On the other hand, since cells underwent apoptosis by overexpression of ANT1 or ANT3, and thus it was not possible to determine whether or not ANT1 or ANT3 is bound to uORF44p in this experiment.

These results of immunoprecipitation suggested that uORF44p binds to at least 4 proteins.

### <<Analysis example>>

In this analysis example, the tissue in which the uORF44p (SEQ ID NO: 1) is expressed in vivo was identified. The uORF44 was an ORF present in the mRNA of ARHGEF 9. The amino acid sequences of human and mouse uORF44p are identical.

First, mRNA expression analysis of ARHGEF9 containing uORF44 was performed by RT-qPCR in 20 kinds of human organs. According to the RefSeq database of NCBI, there are three transcript variants in ARHGEF 9, one of which does not include uORF 44. Therefore, two qPCR primer sets were designed. One (qPCR muORF44-s: GACAGGGAAAGAGAGGGAGAAA (SEQ ID NO: 6), qPCR muORF44-as: GCCTCAGCACTAACGATGGAA (SEQ ID NO: 7)) is to amplify ARHGEF9 including uORF44, and the other (QPCR mArhgef 9-s: CGCCATTACATCAAGCACCTC (SEQ ID NO: 8), qPCR mArhgef 9-as: CACGAAGCCCATCTGAAATCTGT (SEQ ID NO: 9)) amplifies all three variants (Figure7A).

As a result of performing qPCR on cDNA of 20 kinds of human organs using these two primer sets, it was found that ARHGEF 9 is strongly expressed in the nervous system such as the brain and spinal cord, and mRNA thereof is hardly expressed in other organs (Figure 7B).

Next, it was examined whether uORF44p was actually translated in the brain. Specifically, protein lysates were prepared by removing the mouse cerebrum, cerebellum, and hippocampus and brainstem from a mouse respectively, and homogenizing them. Then, it was examined whether uORF44p is expressed by western blotting using an anti-uORF44p antibody. As a result, the bands thereof could be detected in the cerebrum and hippocampus (FIG. 8A).

In order to confirm this result, cerebral lysate was immunoprecipitated using an anti-uORF44 antibody, and uORF44 with a high protein concentration was directly sequenced by mass spectrometry. As a result of immunoprecipitation, enrichment of protein considered to be uORF44 could be detected, and further the band thereof could be detected by SYPRO Ruby staining (FIG. 8B, C).

### <<Example 2>>

In this example, a polyclonal antibody (rabbit antiserum) against uORF44p was prepared.

The partial peptide "MDSLTEQRLTSPNLPAPHLEHYSVLH" of uORF44p was synthesized and used as an immunogen for crosslinking with a carrier protein. KLH was used as a carrier protein.

2 mg of the immunizing antigen was dissolved in 500 µL of PBS (phosphate buffer pH 7.4) and mixed thoroughly with 500 µL of Freund's complete adjuvant to prepare an emulsion. This emulsion was inoculated into several subcutaneous sites of rabbits (Japanese White Rabbit, female). After 3 weeks, half of the synthetic peptide (1 mg) was thoroughly mixed with Freund's incomplete adjuvant to prepare an emulsion, and the emulsion was inoculated into several subcutaneous sites thereof (second inoculation). After 3 weeks, the same amount of synthetic peptide (1 mg) as that of the second inoculation was thoroughly mixed with Freund's complete adjuvant to prepare an emulsion, which was inoculated into two sites of the lumbar muscle of the rabbit (third inoculation). One week after the third inoculation, a large amount of blood was collected from the ear vein, to obtain about 40 mL of serum. Approximately 40 mL of blood serum was collected once a week until 4 weeks thereafter, and the obtained rabbit serum was purified and used.

### <<Example 3>>

### (1) Preparation of knockout mouse

In this example, knockout mice of uORF44p were prepared. The knockout mice were prepared by replacing the ATG codon in uORF44 with TAG of the stop codon so as not to change the expression level of mRNA encoding Arhgef9 containing uORF44. In the case of uORF44, in addition to its start codon, there are two ATG codons encoding methionine in its uORF, and thus these three ATGs were converted to TAG. A schematic view of the targeting vector is shown in Figure 9. The targeting vector used has loxP sequences on both sides of the neomycin resistance gene and sequences homologous to the genome outside the loxP sequences. The homologous region on the 3 'side completely matches the genomic sequence, whereas in the homologous region on the 5' side, all three ATGs of uORF 44 have been changed to TAG. This vector was introduced into mouse ES cells by electroporation. As shown in FIG. 9, the start codon of uORF 44 is replaced by homologous recombination with the target genomic region, and a cell having a genome into which the neomycin resistance gene (Neo) is inserted is produced. Three ES cell lines that underwent homologous recombination could be established by drug selection of ES cells with neomycin. The neomycin gene sandwiched between loxPs with the same orientation was excised by introducing Cre protein into these cell lines using electroporation, and whereby a knockout ES cell having the genome described in FIG. 9 was established. In this targeting vector, one loxP sequence remains in the intron of Arhgef 9 even after removal of the neomycin gene, as shown in FIG. 9. As a control ES cell to investigate the influence of the loxP residual, knockout ES cells in which uORF44 is a wild type (i.e. a type that did not convert the start codon into a stop codon) and loxP is inserted at the same genomic locus were prepared.

Chimeric mice, heterozygous mice, and homozygous KO mice were prepared by a conventional method from the obtained uORF44_KO_ES cells.

### (2) Translation analysis of uORF44 and mORF of Arhgef9 in knockout mice

Translation of uORF44 and mORF of Arhgef9 in the obtained uORF44 knockout mouse was examined. In particular, the translation and expression thereof in the hippocampus of the knockout mouse was examined by western blotting. As a result, uORF44p was not expressed at all in the knockout mice (Figure 10). Further, expression of uORF44p was detected in the wild-type (WT) mouse in the olfactory bulb, but expression was completely inhibited in the knockout mouse.

On the other hand, there was no significant difference in translation level and expression level of Arhgef 9 protein from mORF in wild-type mouse and knockout mouse. It was the same trend in the cerebellum where only Arhgef 9 is translated, that is, the Arhgef 9 translation amount in the cerebellum was not significantly different between wild-type mouse and knockout mouse.

### (3) Phenotype analysis of knockout mouse

Knockout mice got pregnant and gave birth normally, but many pups died before weaning after birth. As a result of the behavioral analysis of knockout mice, it was found that knockout mice show remarkable abnormality in childcare behavior.

Even wild-type female mice without childbirth will make nests, when living with pups for a certain period of time, and then the female mice show behavior to retrieve pups to their nests (retrieving) and behavior to overhang pups (crouching over). However, even though the knockout mice were placed in the same cage together with the pups at the same time, the above-mentioned childcare behavior was not observed at all (FIG. 11).

### <<Example 4>>

In this example, ouORF encoding a functional protein of human mRNA was identified. The procedures of Example 1 (1) to (8) were repeated, except that the research subjects were expanded to upstream ORFs (ouRNAs) partially overlapping with mORF.

As a result, it was suggested that a protein (hereinafter referred to as ouORF7p) derived from a uORF (hereinafter referred to as ouORF7) binds to one kind of protein (FIG. 12).

This binding protein candidate was excised from a SDS-PAGE gel and the protein was identified by mass spectrometry. It was peroxiredoxin.

In order to confirm the results of the immunoprecipitation experiment, western blotting was performed using an antibody against peroxiredoxin. The procedure described in paragraph "(5) western blotting" was repeated except that an anti-peroxiredoxin antibody (Cell Signaling Technology) was used as the primary antibody. From the above results, it was suggested that uORF7p was bound to peroxiredoxin.

### <<Example 5>>

In this example, ouORF encoding a functional protein of human mRNA was identified. The procedures of Example 1 (1) to (8) were repeated except that the research subjects were expanded to upstream ORFs (ouRNAs) partially overlapping with mORF.

As a result, it was suggested that a protein (hereinafter referred to as ouORF21p) derived from a uORF (hereinafter referred to as ouORF21) binds to two kinds of proteins (FIG. 13).

This binding protein candidate was excised from a SDS-PAGE gel and the protein was identified by mass spectrometry. It was C1QBP.

In order to confirm the results of the immunoprecipitation experiment, western blotting was performed using an antibody against C1QBP. The procedure described in paragraph "(5) western blotting" was repeated except that the anti-C1QBP antibody (Santa Cruz) was used as the primary antibody. From the above results, it was suggested that uORF21p was bound to Santa Cruz.

### INDUSTRIAL APPLICABILITY

In the method for identifying a physiologically active protein of the present invention, a physiologically active protein other than the main protein encoded by messenger RNA can be identified. The physiologically active protein of the present invention is thought to be a protein related to behavior and can be used for the development of medicine for mental diseases. Further, the knockout mouse of the physiologically active protein can be used as a model mouse for mental diseases. Furthermore, a compound for preventing or treating a mental disorder on the basis of childcare behavior can be screened by administering a candidate drug to the knockout mouse.

Although the present invention has been described with reference to specific embodiments, various changes and modifications obvious to those skilled in the art are possible without departing from the scope of the appended claims.

## Claims

1. A method for identifying a physiologically active protein, wherein an ORF which encodes a protein having a physiological activity, other than a main open reading frame (ORF) is identified in eukaryotic mRNA, comprising the steps of:
(1) introducing an expression vector incorporating a candidate ORF to cells, and culturing the introduced cells, wherein a start codon of the candidate ORF is AUG or non-AUG in which any one base of AUG is different from AUG,
(2) detecting a protein bound to a candidate protein translated from the candidate ORF, from the cultured cells, and
(3) determining a candidate protein in which another protein bound to the candidate protein is detected as a physiologically active protein.

2. The method for identifying a physiologically active protein according to claim 1, wherein a proteasome inhibitor or a lysosome inhibitor is added to a cell culture medium in the cell culture step (1).

3. The method for identifying a physiologically active protein according to claim 1 or 2, further comprising a step of:
detecting an expression of the candidate protein translated from a candidate ORF in advance and selecting a candidate ORF having a large amount of expression, before the binding protein detection step (2).

4. The method for identifying a physiologically active protein according to any one of claims 1 to 3, wherein the candidate ORF satisfies one or more conditions selected from the group consisting of:
(a) the start codon of the candidate ORF is present on the 5 'UTR side from the start codon of the main ORF,
(b) a protein encoded by a candidate ORF is 10 amino acids or more,
(c) a candidate ORF is an ORF other than the main ORF in mRNA of one eukaryotic organism, and an amino acid sequence translated from the candidate ORF has 50% or more identity to an amino acid sequence translated from an ORF other than the main ORF in mRNA of one or more other eukaryotic organisms,
(d) the start codon of the candidate ORF is AUG; and
(e) peptides expressed in a cell of the eukaryotic organism are analyzed by mass analysis, and an ORF of an amino acid sequence obtained by using the molecular weight of the obtained peptide is a matched one retrieved from a database of the candidate ORFs.

5. The method for identifying a physiologically active protein according to any one of claims 1 to 4, wherein a method for detecting a binding protein in the binding protein detection step (2) is selected from the group consisting of an immunoprecipitation method, yeast two-hybrid method, a protein array method, a label method using peroxidase, and a BioID method.

6. The method for identifying a physiologically active protein according to any one of claims 1 to 5, wherein the eukaryotic organism is a mammal.

7. (1) a protein consisting of an amino acid sequence of SEQ ID NO:1,
(2) a protein comprising an amino acid sequence of SEQ ID NO:1, and exhibiting a function of suppressing abnormality of mental activity on the basis of childcare behavior,
(3) a protein consisting of an amino acid sequence in which one or plural amino acids are deleted, substituted, inserted and/or added at one or plural positions in the amino acid sequence of SEQ ID NO:1, and exhibiting a function of suppressing abnormality of mental activity on the basis of childcare behavior, or
(4) a protein comprising an amino acid sequence in which one or plural amino acids are deleted, substituted, inserted and/or added at one or plural positions in the amino acid sequence of SEQ ID NO:1, and exhibiting a function of suppressing an abnormality of mental activity on the basis of childcare behavior.

8. (1) a protein consisting of an amino acid sequence of SEQ ID NO:10 or 11,
(2) a protein comprising an amino acid sequence of SEQ ID NO: 10 or 11, and having a binding ability with the peroxiredoxin 1,
(3) a protein consisting of an amino acid sequence in which one or plural amino acids are deleted, substituted, inserted and/or added at one or plural positions in the amino acid sequence of SEQ ID NO:10 or 11, and having a binding ability with the peroxiredoxin 1, or
(4) a protein comprising an amino acid sequence in which one or plural amino acids are deleted, substituted, inserted and/or added at one or plural positions in the amino acid sequence of SEQ ID NO:10 or 11, and having a binding ability with the peroxiredoxin 1.

9. (1) a protein consisting of an amino acid sequence of SEQ ID NO:14 or 15,
(2) a protein comprising an amino acid sequence of SEQ ID NO: 14 or 15, and having a binding ability with the Q Subcomponent Binding Protein,
(3) a protein consisting of an amino acid sequence in which one or plural amino acids are deleted, substituted, inserted and/or added at one or plural positions in the amino acid sequence of SEQ ID NO: 14 or 15, and having a binding ability with the Q Subcomponent Binding Protein, or
(4) a protein comprising an amino acid sequence in which one or plural amino acids are deleted, substituted, inserted and/or added at one or plural positions in the amino acid sequence of SEQ ID NO: 14 or 15, and having a binding ability with the Q Subcomponent Binding Protein.

10. A polynucleotide encoding the protein according to any one of claims 7 to 9.

11. An expression vector comprising the polynucleotide according to claim 10.

12. A transformant comprising the polynucleotide according to claim 10.

13. An antibody or a fragment thereof, which binds to the protein according to any one of claims 7 to 9.

14. A knockout non-human animal or cell, wherein an expression of a gene encoding the protein according to claim 7 is partially or completely suppressed.

15. A method for screening a compound that suppresses abnormality of mental activity on the basis of childcare behavior, **characterized by** administering a candidate compound
to the knockout non-human animal or cell according to claim 14.


SEQUENCE LISTING

<110>  Tokyo Institute of Technology
       LifeIS INC.

<120>  A method for identifying bioactive proteins and a bioactive
       protein obtained by the method

<130>  TIT-993

<150>  JP 2015-180831
<151>  2015-09-14

<160>  17

<170>  PatentIn version 3.5

<210>  1
<211>  59
<212>  PRT
<213>  Homo sapiens

<400>  1

Met Asp Ser Leu Thr Glu Gln Arg Leu Thr Ser Pro Asn Leu Pro Ala
1               5                   10                  15

Pro His Leu Glu His Tyr Ser Val Leu His Cys Thr Met Thr Leu Asp
            20                  25                  30

Val Gln Thr Val Val Val Phe Ala Val Ile Val Val Leu Leu Leu Val
        35                  40                  45

Asn Val Ile Leu Met Phe Phe Leu Gly Thr Arg
    50                  55

<210>  2
<211>  180
<212>  DNA
<213>  Homo sapiens

<400>  2
atggacagtc tgacagaaca gagactgaca tctcccaatc tgccggcccc ccacctggaa      60

cactacagtg ttctgcattg caccatgacc ctggatgtgc aaactgtagt cgtttttgcc     120

gtgattgtag tcctcctgct tgtcaatgtc atactcatgt ttttcctggg aacgcgctga     180

<210>  3
<211>  180
<212>  DNA
<213>  Mus musculus

<400>  3
atggacagtc tgacagaaca gagattgaca tctcccaatc tgccggcccc ccacctggaa      60

cactacagtg ttctgcattg caccatgacc ctggatgtgc aaactgtagt cgtttttgcc     120

gtgattgtag tcctcctgct tgtcaatgtc atactcatgt ttttcttggg aacgcgctga     180

<210>  4
<211>  32
<212>  DNA
<213>  Artificial Sequence

<220>
<223>  FLAG-uORF44 sense primer

<400>  4
ggaggatccg gcgacagtct gacagaacag ag                                    32

<210>  5
<211>  32
<212>  DNA
<213>  Artificial Sequence

<220>
<223>  FLAG-uORF44 antisense primer

<400>  5
ccggaattct tatcagcgcg ttcccaggaa aa                                    32

<210>  6
<211>  22
<212>  DNA
<213>  Artificial Sequence

<220>
<223>  qPCR muORF44-s

<400>  6
gacagggaaa gagagggaga aa                                               22

<210>  7
<211>  21
<212>  DNA
<213>  Artificial Sequence

<220>
<223>  qPCR muORF44-as

<400>  7
gcctcagcac taacgatgga a                                                21

<210>  8
<211>  21
<212>  DNA
<213>  Artificial Sequence

<220>
<223>  qPCR mArhgef9-s

<400>  8
cgccattaca tcaagcacct c                                                21

<210>  9
<211>  23
<212>  DNA
<213>  Artificial Sequence

<220>
<223>  qPCR mArhgef9-as

<400>  9
cacgaagccc atctgaaatc tgt                                              23

<210>  10
<211>  56
<212>  PRT
<213>  Homo sapiens

<400>  10

Met Ser Ala Gly Arg Ala Ser Gly Ala Leu Ser Arg Thr Arg Thr Pro
1               5                   10                  15

Leu Glu Pro Glu Pro Gln Gln Lys Pro Glu Pro Glu Pro Asn His Arg
            20                  25                  30

Cys Ser Pro Leu Asn Pro Gly Gly Ala Leu Ala Arg Ala Arg Pro Pro
        35                  40                  45

Gly Pro His Val Gly Thr Thr Ala
    50                  55

<210>  11
<211>  58
<212>  PRT
<213>  Mus musculus

<400>  11

Met Ser Ser Gly Arg Ala Ser Gly Ala Leu Ser Arg Thr Arg Thr Pro
1               5                   10                  15

Leu Glu Pro Gly Pro Glu Pro Glu Pro Glu Pro Glu Thr Glu Pro Asn
            20                  25                  30

His Arg Cys Ser Pro Leu Asn Pro Gly Gly Ala Leu Ala Arg Ala Arg
        35                  40                  45

Pro Pro Gly Pro His Val Gly Thr Thr Ala
    50                  55

<210>  12
<211>  171
<212>  DNA
<213>  Homo sapiens

<400>  12

atgagcgccg gaagggcgtc gggggccctg agccgcacta ggacgcccct ggagccggaa      60

ccccagcaga agccggaacc agaaccaaat caccgctgca gccccctaaa cccaggaggc     120

gccctggccc gcgctcgccc cccagggcct catgtcggaa ccacagcctg a              171

<210>  13
<211>  177
<212>  DNA
<213>  Mus musculus

<400>  13
atgagctccg ggagggcgtc gggggccctg agccggacga ggacgcccct ggagccggga      60

cccgaaccgg agccggagcc ggagacagaa ccaaaccacc gctgcagccc actaaaccca     120

ggaggcgccc tggcccgcgc tcgcccccca gggcctcatg tcggaaccac agcctga        177

<210>  14
<211>  49
<212>  PRT
<213>  Homo sapiens

<400>  14

Met Arg Lys Gly Ala Arg Arg Leu Cys Pro Ala Ala Arg Val Ser Met
1               5                   10                  15

Glu Arg Arg Arg Pro Arg Leu Leu Arg Arg Arg Leu Arg Gln Trp Arg
            20                  25                  30

Trp Arg Pro Phe Leu Lys Lys Trp Thr Ser Ser Pro Pro His Thr Gly
        35                  40                  45

Gly

<210>  15
<211>  49
<212>  PRT
<213>  Mus musculus

<400>  15

Met Arg Arg Gly Ala Arg Arg Leu Cys Pro Ala Ala Arg Val Ser Met
1               5                   10                  15

Glu Arg Arg Arg Pro Arg Leu Leu Arg Arg Thr Leu Arg Gln Trp Arg
            20                  25                  30

Trp Arg Pro Ser Pro Met Lys Trp Thr Ser Ser Pro Pro Arg Thr Gly
        35                  40                  45

Gly

<210>  16
<211>  150
<212>  DNA
<213>  Homo sapiens

<400>  16
atgcgcaagg gggcgcgccg cctctgcccc gcggcgaggg tgtctatgga gaggcggcgg      60

ccgcggctgc tgaggcggag gctgaggcag tggcgatggc gccctttcct gaagaagtgg     120

acgtcttcac cgccccacac tggcggatga                                      150


<210>  17
<211>  150
<212>  DNA
<213>  Mus musculus

<400>  17
atgcgcaggg gggcgcgccg cctctgcccc gcggcgaggg tgtctatgga gaggcggcgg      60

ccgcggctgc tgaggcggac gctgaggcag tggcgatggc gcccttcccc gatgaagtgg     120

acgtcttcac cgccccgcac tggcggatga                                      150